# EUROPEAN PATENT APPLICATION

(11) **EP 2 042 872 A1**
(43) Date of publication of application: **01.04.2009**
(21) Application number: 08164425.4
(22) Date of filing: 16.09.2008
(51) Int. Cl.: G01N 33/543

(54) **Sandwich immunoassay and method of detecting an antigen by using the same**

(30) Priority: 28.09.2007 JP 2007255331
(71) Applicant: Sysmex Corporation, Kobe-shi, Hyogo 651-0073 (JP)
(72) Inventor: Fujimoto, Hiroyuki, Kobe-shi Hyogo 651-0073 (JP); Matsue, Miyuki, Kobe-shi Hyogo 651-0073 (JP)
(74) Representative: De Clercq, Ann G. Y.

(57) **Abstract**

The invention provides a sandwich immunoassay method comprising steps of, (a) forming a complex of an antigen and a first antibody by contacting the antigen with the first antibody which recognizes the antigen and is labelled by a detectable labelling substance; and (b) fixing the complex formed in the step (a) to a solid phase by using a second antibody which recognizes the antigen and is capable of binding to the solid phase, as well as a method for detecting an antigen in an analyte by using a method of sandwich immunoassay.

## Description

### FIELD OF THE INVENTION

The present invention relates to a sandwich immunoassay capable of highly sensitive detection of an antigen occurring in only a small amount in a sample, and a method of detecting an antigen by using the same.

### BACKGROUND

As a method of detecting a substance (antigen) in a sample, a sandwich method is known. In the sandwich method, an antigen is detected by "sandwiching" the antigen between two different antibodies recognizing non-overlapping epitopes of the antigen. In the usual sandwich method, an antigen is first bound to a first antigen-specific antibody that was bound to a solid phase. An antigen/first antibody complex is thereby formed on the solid phase. Then, a second antigen-specific antibody labelled with a detectable labelling substance is bound to the antigen on the solid phase thereby detecting the antigen.

For example, JP-A 57-074663 discloses a method of measuring TSH by using a sandwich enzyme immunoassay by allowing a TSH-containing sample to act on a solid phase to which an antibody was bound, and then allowing an enzyme-labelled antibody to act thereon. That is, JP-A 57-074663 discloses the conventional sandwich method wherein an antibody that was bound to a solid phase is used to fix an antigen to the solid phase and then an enzyme-labelled antibody is allowed to act thereon to detect the antigen.

In the conventional sandwich method, however, it occurs that when the affinity for its antigen of an antibody used in measurement is not so high, the problem arises that when an antigen occurs in only a small amount in a sample, highly sensitive measurement is difficult.

### SUMMARY

The scope of the present invention is defined solely by the appended claims, and is not affected to any degree by the statements within this summary.

The object of the present invention is to provide a sandwich immunoassay capable of highly sensitive detection of an antigen and a method of detecting an antigen by using the same.

### DETAILED DESCRIPTION OF THE EMBODIMENT

In this specification, the "sandwich immunoassay" means a method of detecting an antigen by two antibodies, that is, an antibody on a solid phase and an antibody labelled with a labelling substance.

The sandwich immunoassay of the present invention comprises (a) a step of contacting an antigen with a first antibody recognizing the antigen and being labelled with a detectable labelling substance, to form an antigen-first antibody complex.

Although the antigen is not particularly limited as long as it is a substance intended to be detected, the antigen is preferably thyroid stimulation hormone (TSH).

Diagnosis of thyroid diseases such as Graves' disease is conducted by examining whether the amount of TSH in a blood sample is reduced. However, the amount of TSH is low even in blood from healthy human subjects. Further, the diagnosis of thyroid diseases requires detection of a reduction in the amount of TSH. By using the sandwich immunoassay of the present invention, TSH can be highly sensitively measured.

The antigen may be an antigen contained in an analyte. The analyte is not particularly limited as long as it is a sample not inhibiting the sandwich immunoassay. For example, blood can be mentioned as the analyte containing TSH as an antigen. The blood includes whole blood, plasma and serum.

A first antibody recognizing the antigen may be a monoclonal antibody or a polyclonal antibody. Particularly, a monoclonal antibody is preferable. The class of the first antibody can be an antibody class used ordinarily in the art. Examples of these classes include IgG, IgM etc. The first antibody includes an antibody fragment and its derivative if it can recognize an intended antigen. The antibody fragment includes a Fab fragment, F(ab')2 fragment, Fab' fragment and sFv fragment.

For example, when the antigen is TSH, the first antibody includes Anti-TSH 5409 (manufactured by Medix Biochemica) that is an anti-TSH monoclonal antibody. Anti-TSH 5409 is excellent in specificity and storage stability, but its affinity for TSH is not so high. It follows that when Anti-TSH 5409 is used in the conventional sandwich immunoassay, highly sensitive detection of TSH is difficult. However, TSH can be detected in a highly sensitive manner by using the sandwich immunoassay of the present invention.

The first antibody can be obtained by methods known in the art. Such known methods include a method wherein a mammal such as a horse, bovine, sheep, rabbit, goat, rat or mouse is immunized with an antigen to be detected, serum is collected from the immunized animal, and its antibody is purified from the serum. Such known methods also include a method wherein an antibody-producing cell collected from an immunized mouse is fused with a mouse tumour cell to give a hybridoma from which an antibody is obtained.

The first antibody is labelled with a detectable labelling substance. The labelling substance is not particularly limited as long as it is a labelling substance that can be used in usual immunoassays. Examples of the labelling substance include an enzyme, a fluorescent substance, a radioisotope, etc. The enzyme includes an alkaline phosphatase, peroxidase, glucose oxidase, tyrosinase, acid phosphatase, etc. The fluorescent substance includes fluorescein isothiocyanate (FITC), green fluorescence protein (GFP), luciferin, etc. The radioisotope includes ¹²⁵I, ¹⁴C, ³²P, etc.

The method of labelling an antibody with a labelling substance may be a method used ordinarily in the art. For example, a method wherein an antibody is bound via thiol (-SH group) in the antibody to a labelling substance is known. Specifically, a labelling substance into which a functional group capable of reacting with a thiol group was introduced can be reacted with an antibody to label the antibody with the labelling substance. The functional group includes, for example, a maleimide group.

The sandwich immunoassay of the present invention comprises (b) a step of fixing the complex formed in the step (a) to a solid phase by using a second antibody recognizing the antigen and being capable of binding to the solid phase. That is, the antigen reacts earlier with the first antibody than with the second antibody, to form an antigen-first antibody complex.

By carrying out the reaction in this order, even an antigen occurring in only a trace amount in a sample can be detected in a highly sensitive manner. The time of reaction between the first antibody and an antigen can be prolonged. It follows that even if the affinity of the first antibody for an antigen is low, the first antibody can be sufficiently bound to the antigen. As a result, highly sensitive measurement is feasible in a short measurement time.

The second antibody recognizing the antigen and being capable of binding to the solid phase is an antibody capable of recognizing a different site (epitope) of the antigen from the site recognized by the first antibody.

Like the first antibody, the second antibody may be a monoclonal antibody or a polyclonal antibody. Particularly, a monoclonal antibody is preferred. The class of the second antibody can be an antibody class used ordinarily in the art. Examples of these classes include IgG, IgM, etc. The second antibody includes an antibody fragment and its derivative if it can recognize an intended antigen. The antibody fragment includes a Fab fragment, F(ab')2 fragment, Fab' fragment and sFv fragment. The method of obtaining the second antibody is the same as for the first antibody described above.

When the antigen is TSH, the second antibody is preferably a monoclonal antibody produced by a hybridoma deposited under NITE BP-399. This hybridoma, which was obtained by fusing a spleen cell of a TSH-immunized BALB/C mouse with a mouse myeloma cell (P3-X63-Ag8-653), was deposited under Accession No. NITE BP-399, on August 21, 2007 with NITE International Patent Organisms Depositary, National Institute of Technology and Evaluation (NITE), National Institute of Advanced Industrial Science and Technology, Japan.

The second antibody can be bound to a solid phase. The bond between the second antibody and a solid phase is a bond between a solid phase-binding substance added to the second antibody and a binding substance bound to the solid phase.

The solid phase-binding substance added to the second antibody and the binding substance bound to the solid phase are not particularly limited insofar as they are a combination of substances that can be specifically bound to each other under the conditions where the sandwich immunoassay is conducted. The combination of such substances is for example a combination of biotin and avidin or streptavidin, a hapten and an anti-hapten antibody, nickel and a histidine tag, or glutathione and glutathione-S-transferase. The hapten and anti-hapten antibody include, for example, dinitrophenol (DNP) and an anti-DNP antibody.

The bond between the second antibody and the solid phase is preferably a biotin-avidin bond or a biotin-streptavidin bond. Accordingly, the combination of the solid phase-binding substance added to the second antibody and the binding substance bound to the solid phase is preferably a combination of biotin and avidin or a combination of biotin and streptavidin.

In the combination of the solid phase-binding substance and the binding substance added to the second antibody, either of the two exemplary substances may be bound to the second antibody. Preferably, the solid-phase binding substance contains biotin and the binding substance is avidin or streptavidin.

The method of adding the solid phase-binding substance to the second antibody is known in the art. For example, a solid phase-binding substance having a group that is reactive to a primary amino group (-NH₂ group) or a thiol group occurring on the antibody can be added to the antibody. The group that is reactive to a primary amino group includes a succinimide group (NHS group) etc. The group that is reactive to a thiol group includes a maleimide group etc.

The material of the solid phase is not particularly limited as long as it is a material of a usual solid phase used in immunoassays. Examples of such material include polymer materials such as latex, rubber, polyethylene, polypropylene, polystyrene, a styrene-butadiene copolymer, polyvinyl chloride, polyvinyl acetate, polyacrylamide, polymethacrylate, a styrene-methacrylate copolymer, polyglycidyl methacrylate, an acrolein-ethyleneglycol dimethacrylate copolymer, polyvinylidene difluoride (PVDF), and silicone; agarose; gelatin; red blood cells; and inorganic materials such as silica gel, glass, inert alumina, and magnetic substances. These materials may be used singly or in combination of two or more thereof.

The form of the solid phase is not particularly limited insofar as the solid phase is in the form of a usual solid phase used in immunoassays, for example in the form of a microtiter plate, a test tube, beads, particles, and nanoparticles. The particles include magnetic particles, hydrophobic particles such as polystyrene latex, copolymer latex particles having hydrophilic groups such as an amino group and a carboxyl group on the surfaces of the particles, red blood cells and gelatin particles.

The solid phase is more preferably magnetic particles. The magnetic particles are those particles that contain a magnetic material as a base material. Such magnetic particles are known in the art. The base materials known in the art includes, for example, those using Fe₂O₃, Fe₃O₄, cobalt, nickel, ferrite, magnetite etc. For binding a protein or the like to the surface of a magnetic particle, the magnetic particle is more preferably that which comprises a base material coated thereon with a polymer or the like.

A method of binding the binding substance to the solid phase is known in the art. This fixation can be carried out for example by a physical adsorption method, a covalent bonding method, an ionic bonding method, or a combination thereof. For example, when the binding substance is avidin or streptavidin, a solid phase to which biotin was bound can be used to fix avidin or streptavidin to the solid phase.

The step (b) is conducted preferably by any one of the following four steps:
(b1) The complex formed in the step (a) is contacted with the second antibody and a solid phase;
(b2) The complex formed in the step (a) is contacted with a solid phase, and then the second antibody is added in the presence of the complex and the solid phase;
(b3) The complex formed in the step (a) is contacted with the second antibody, and a solid phase is added in the presence of the complex and the second antibody; and
(b4) The second antibody is contacted with a solid phase, and then the complex formed in the step (a) is added in the presence of the second antibody and the solid phase.

The reactions estimated to occur in these cases are as follows:
Case (b1): Case where the complex formed in the step (a) is contacted with the second antibody and a solid phase:
   The antigen-first antibody complex formed in the step (a) is contacted with the second antibody and the solid phase simultaneously. By doing so, the second antibody is bound to the antigen-first antibody complex to form a first antibody-antigen-second antibody
   complex which is then bound to the solid phase, whereby the first antibody-antigen-second antibody complex can be formed on the solid phase. Also, the second antibody can be bound to the solid phase and then bound to the antigen-first antibody complex to form a first antibody-antigen-second antibody complex on the solid phase.
Case (b2): Case where the complex formed in the step (a) is contacted with a solid phase, and then the second antibody is added in the presence of the complex and the solid phase:
   The antigen-first antibody complex formed in the step (a) is contacted with the solid phase. In this case, the antigen-first antibody complex does not have a site for binding to the solid phase. Accordingly, no reaction occurs between the antigen-first antibody and the solid phase. Then, the second antibody is provided. The second antibody is thereby bound to the antigen-first antibody complex and then bound to the solid phase, whereby a first antibody-antigen-second antibody complex can be formed on the solid phase. Also, the second antibody can be bound to the solid phase and then bound to the antigen-first antibody complex to form a first antibody-second antibody complex on the solid phase.
Case (b3): Case where the complex formed in the step (a) is contacted with the second antibody, and a solid phase is added in the presence of the complex and the second antibody:
   The antigen-first antibody complex formed in the step (a) is contacted with the second antibody, thereby forming a first antibody-antigen-second antibody complex. Thereafter, the solid phase is provided, whereby a first antibody-antigen-second antibody complex can be fixed to the solid phase.
Case (b4): Case where the second antibody is contacted with a solid phase, and then the complex formed in the step (a) is added in the presence of the second antibody and the solid phase:
   By contacting the second antibodies with the solid phase, all or some of the second antibodies are bound to the solid phase. Then, the complex formed in the step (a) is added. The second antibody bound to the solid phase can thereby be bound to the complex to form a first antibody-antigen-second antibody complex on the solid phase. Also, the second antibody not bound to the solid phase is bound to the antigen-first antibody complex to form a first antibody-antigen-second antibody complex, and then this complex is bound via the second antibody to the solid phase, whereby a first antibody-antigen-second antibody complex can also be formed on the solid phase.
   Preferably the sandwich immunoassay of the present invention further comprises (c) a step of measuring the labelling substance of the first antibody fixed via the second antibody and the antigen to the solid phase, after formation of the first antibody-antigen-second antibody complex on the solid phase.
   The method of measuring the labelling substance of the first antibody can be appropriately selected depending on the type of the labelling substance. For example, when the labelling substance is a radioisotope, a method of measuring radioactivity by using a conventionally known apparatus such as a scintillation counter can be used. When the labelling substance is a fluorescent substance, a method of measuring fluorescence by using a conventionally known apparatus such as a luminometer can be used.
   When the labelling substance is an enzyme, a method of measuring luminescence or coloration by reacting an enzyme substrate with the enzyme can be used. The substrate that can be used for the enzyme includes a conventionally known luminescent substrate, colorimetric substrate, or the like. When an alkaline phosphatase is used as the enzyme, its substrate includes chemilumigenic substrates such as CDP-star (registered trademark) (4-chloro-3-(methoxyspiro{1,2-dioxetane-3,2'-(5'-chloro) tricyclo[3.3.1.1^{3.7}] decane}-4-yl) disodium phenylphosphate) and CSPD (registered trademark) (3-(4-methoxyspiro{1,2-dioxetane-3,2-(5'-chloro) tricyclo[3.3.1.1^{3.7}] decane}-4-yl) disodium phenylphosphate) and colorimetric substrates such as p-nitrophenyl phosphate, 5-bromo-4-chloro-3-indolyl-phosphoric acid (BCIP), 4-nitro blue tetrazolium chloride (NBT), and iodonitro tetrazolium (INT). These luminescent or colorimetric substrates can be detected by a conventionally known spectrophotometer, luminometer, or the like.
   The sandwich immunoassay of the present invention may comprise a washing step after step (b). By washing, the unreacted first antibody can be removed. For example, when the solid phase comprises magnetic particles, a washing substance or bufferis added to the magnetic particles-containing sample obtained in the steps (a) and (b) and then stirred. Thereafter, the magnetic particles are adsorbed with magnetism, and in this state, the washing substance can be removed to remove the unreacted first antibody. The washing substance that can be used herein may be one known in the art. Examples of the wash that can be used in this step include 2-[N-morpholino]ethanesulfonate buffer (MES), phosphate buffered saline (PBS), etc. The pH of the buffer is preferably from about pH 6.0 to about pH 10.0. The buffer may contain a surfactant such as Tween 20.

100 to 700 µL of the washing substance can be added to the sample and subjected to magnetic separation. This washing operation can be carried out 4 times in total.

The sandwich immunoassay can be carried out under conditions for usual immunoassays. The conditions for usual immunoassays are the conditions under which the pH is about 6.0 to 10.0 and the temperature is about 30 to 45°C.

The pH can be regulated with a buffer such as phosphate buffered saline (PBS), a triethanolamine hydrochloride buffer (TEA), a Tris-HCl buffer or the like. The buffer may contain components used in usual immunoassays, such as a surfactant, a preservative and serum proteins.

The time of contacting the respective components in each of the respective steps can be suitably established depending on the antigen to be measured, the antibody to be used, and the type of the solid phase or the like.

The amounts of the first antibody, the second antibody and the solid phase to be used can also be suitably established depending on the antigen to be measured, the antibody to be used, and the type of the solid phase or the like.

The method for detecting an antigen according to the present invention is a method for detecting an antigen in an analyte. The method for detecting an antigen according to the present invention comprises the following steps:
(A) mixing an analyte with a first antibody recognizing the antigen and being labelled with a detectable labelling substance;
(B) adding a solid phase and a second antibody recognizing the antigen and being capable of binding to the solid phase to the mixture obtained in the step (A); and
(C) detecting the labelling substance of the first antibody that was bound to the solid phase.

Step (A) corresponds to step (a) in the sandwich immunoassay described above. That is, when the antigen as an object of detection is present in an analyte, the analyte is contacted with a first antibody. By doing so, an antigen-first antibody complex is formed.

Step (B) corresponds to step (b) in the sandwich immunoassay described above. That is, when an antigen-first antibody complex is formed in step (A), the antigen-first antibody complex is fixed via the second antibody to the solid phase.

Step (B) is conducted preferably by any one of the following four steps:
(B1) A solid phase and a second antibody are simultaneously added to the mixture obtained in step (A);
(B2) A solid phase is added to the mixture obtained in step (A), and then a second antibody is added;
(B3) A second antibody is added to the mixture obtained in step (A), and then a solid phase is added; and
(B4) A solid phase is mixed with a second antibody, and then a mixture of the solid phase and the second antibody is added to the mixture obtained in step (A).

The reactions in steps (B1) to (B4) are the same as those in steps (b1) to (b4) described above. That is, the reaction in step (B1) corresponds to the reaction in step (b1). The reaction in step (B2) corresponds to the reaction in step (b2). The reaction in step (B3) corresponds to the reaction in step (b3). The reaction in step (B4) corresponds to the reaction in step (b4).

Step (C) corresponds to step (c) in the sandwich immunoassay described above. When the antigen as an object of detection is present in an analyte, a first antibody-antigen-second antibody complex is fixed to a solid phase. Accordingly, the antigen in the analyte can be detected by detecting the labelling substance of the first antibody fixed via the second antibody and the antigen to the solid phase.

Preferably the sandwich immunoassay of the present invention further comprises a step of washing the solid phase after step (B). By washing, the unreacted first antibody can be removed from the mixture obtained in step (B). The washing substance used in this washing step is the same as the above-mentioned washing substances. In the mixture from which the unreacted first antibody is thereby removed, the labelling substance of the first antibody fixed via the second antibody and the antigen to the solid phase can be detected in step (C).

### EXAMPLES

Hereinafter, the present invention is described in more detail by reference to the Examples, but the present invention is not limited to the Examples that follow. -Preparation of a biotin-labelled anti-TSH monoclonal antibody-

The monoclonal antibody used as a biotin-labelled anti-TSH monoclonal antibody was prepared by the following method.

10 to 100 µg TSH was dissolved in 0.5 ml PBS, and 0.5 ml FCA (DIFCO) was added as adjuvant thereto to prepare a water-in-oil antigen for basic immunization. The antigen for basic immunization was administered intraperitoneally to a BALB/C mouse. The intraperitoneal administration was carried out several times every 2 to 4 weeks. A booster antigen prepared by dissolving 100 µg TSH in 1 ml PBS was used.

Spleen cells from the immunized BALB/C mouse, and mouse myeloma cells (P3-X63-Ag8-653), were mixed at a ratio of 10 : 1. Then, 1 ml of 50% polyethylene glycol was added dropwise to a mixture of the cells and stirred for 1 minute at room temperature. While the mixture was stirred, 5 to 10 ml MEM medium was gradually added thereto in 2 to 5 minutes. After washing, the fused cells were suspended at a density of 3.5×10⁶ cells/ml in a HAT medium and then pipetted in a volume of 100 µl/well onto a 96-well micro-culture plate. Growth of the hybridomas was observed, a HAT medium was added at an appropriate time, and they were cultured for about 2 weeks.

Each of their culture supernatants was added to an antigen-conjugated plate (TSH-β chain-conjugated plate), and a hybridoma producing an anti-TSH monoclonal antibody recognizing a TSH-β chain was selected by ELISA (enzyme-linked immunosorbent assay). The selected hybridoma was cloned to establish a cell strain T2-194 (NITE BP-399).

T2-194 was cultured, and its supernatant was purified with protein A to give a monoclonal antibody. The resulting monoclonal antibody was reacted in a usual manner with biotin-maleimide to give a biotin-labelled anti-TSH monoclonal antibody.

### Example 1

Thyroid stimulation hormone (TSH) was measured by the sandwich immunoassay of the present invention in the following procedures.

In the following procedures, the antibody was diluted with an antibody buffer (0.1 M MES (2-morpholinoethanesulfonate, pH 6.0, 1% bovine serum albumin, 0.15 M NaCl, 0.1% sodium azide).
1) 30 µL of an alkaline phosphatase (ALP)-labelled anti-TSH monoclonal antibody (0.4 U/mL, manufactured by Medix Biochemica) as a labelling substance-labelled first antibody, and 30 µL of a TSH antigen solution (concentration: 0 or 2 µIU/mL, manufactured by Fitzgerald) as a sample, were pipetted into each well of a reaction vessel and reacted with each other at 42°C for 2 minutes.
2) 30 µL of streptavidin magnetic particles (a suspension of 10 mg /mL particles having an average size of 2 µm in 20 mM sodium phosphate buffer, pH 7.5) were added to each well and reacted at 42°C for 2.5 minutes.
3) As a second antibody capable of binding to the solid phase, 30 µL of the biotin-labelled anti-TSH monoclonal antibody (10 µg/mL) obtained in the preparation of the biotin-labelled anti-TSH monoclonal antibody as described above was pipetted into each well, reacted at 42°C for 2.5 minutes, and separated by magnetism.
4) 100 to 700 µL of a wash (0.01 M MES, pH 6.5, 0.1% sodium azide) was added to each well, stirred, and separated by magnetism. This operation was carried out 4 times in total.
5) 50 µL of a dispersing liquid (0.01 M MES, pH 6.5, 0.1% sodium azide) was added to each well and stirred to disperse the streptavidin magnetic particles.
6) 100 µL of a luminescent substrate solution (CDP-Star, Applied Biosystems) was added to each well, stirred, reacted at 42°C for 5 minutes, and measured for its luminescence intensity with a luminometer.

### Comparative Example 1

TSH was measured by the conventional sandwich immunoassay in the following procedures.
1) 30 µL of the biotin-labelled anti-TSH monoclonal antibody (10 µg/mL) as an antibody capable of binding to a solid phase, which was obtained in the preparation of the biotin-labelled anti-TSH monoclonal antibody as described above, and 30 µL of a TSH antigen solution (concentration: 0 or 2 µIU/mL) as a sample, were pipetted into each well of a reaction vessel and reacted with each other at 42°C for 2 minutes.
2) 30 µL of streptavidin magnetic particles (a suspension of 10 mg /mL particles having an average size of 2 µm in 20 mM sodium phosphate buffer, pH 7.5) were added to each well and reacted at 42°C for 2.5 minutes.
3) An alkaline phosphatase (ALP)-labelled anti-TSH monoclonal antibody (0.4 U/mL, manufactured by Medix Biochemica) was added, reacted at 42°C for 2.5 minutes, and separated by magnetism.
4) 100 to 700 µL of a washing substance was added to each well, stirred, and separated by magnetism. This operation was carried out 4 times in total.
5) 50 µL of a dispersing liquid was added to each well and stirred to disperse the streptavidin magnetic particles.
6) 100 µL of a luminescent substrate solution (CDP-Star, Applied Biosystems) was added to each well, stirred, reacted at 42°C for 5 minutes, and measured for its luminescence intensity with a luminometer.

The luminescence intensity obtained by using the TSH-containing sample, and the ratio of the luminescence intensity obtained by using the TSH-containing sample to the luminescence intensity obtained by using the TSH-free sample (signal/noise ratio (S/N ratio)), are shown in Table 1.

**Table 1**

| TSH Concentration (µIU/mL) | Example 1 | | Comparative Example 1 | |
|---|---|---|---|---|
| | Luminescence Intensity | S/N Ratio | Luminescence Intensity | S/N Ratio |
| 0 | 1,589 | 807 | 1,656 | 193 |
| 2 | 1,283,000 | | 319,500 | |

From Table 1, it was revealed that when the sandwich immunoassay of the present invention is used, the luminescence intensity obtained by using the TSH-containing sample is increased, and the S/N ratio is increased.

The foregoing detailed description and examples have been provided by way of explanation and illustration, and are not intended to limit the scope of the appended claims. Many variations in the presently preferred embodiments will be obvious to one of ordinary skill in the art, and remain within the scope of the appended claims and their equivalents.

## Claims

1. A sandwich immunoassay method, comprising the following steps :
(a) forming a complex of an antigen and a first antibody by contacting the antigen with the first antibody which recognizes the antigen and which is labelled by a detectable labelling substance; and
(b) fixing the complex formed in step (a) to a solid phase by using a second antibody which recognizes the antigen and which is capable of binding to the solid phase.

2. The method according to claim 1, wherein the antigen is thyroid stimulation hormone.

3. The method according to claim 1 or 2, wherein step (b) is performed so as to contact the complex formed in the step (a), the second antibody and the solid phase.

4. The method according to claim 1 or 2, wherein step (b) is performed so as to add the second antibody in the presence of the complex formed in the step (a) and the solid phase after contacting the complex with the solid phase.

5. The method according to claim 1 or 2, wherein step (b) is performed so as to add the solid phase in the presence of the complex formed in the step (a) and the second antibody after contacting the complex with the second antibody.

6. The method according to claim 1 or 2, wherein step (b) is performed so as to add the complex formed in the step (a) in the presence of the second antibody and the solid phase after contacting the second antibody with the solid phase.

7. The method according to any one of claims 1 to 6 further comprising a step of measuring the labelling substance of the first antibody bound to the solid phase via the second antibody and the antigen after the step (b).

8. A method for detecting an antigen in an analyte, comprising the following steps :
(A) mixing the analyte and a first antibody which recognizes the antigen and which is labelled by a detectable labelling substance;
(B) adding a solid phase and a second antibody which recognizes the antigen and which is capable of binding to the solid phase to the mixture obtained in step (A);
(C) detecting the labelling substance of the first antibody bound to the solid phase via the second antibody and the antigen.

9. The method according to claim 8, wherein the antigen is thyroid stimulation hormone.

10. The method according to claim 11, wherein step (B) is performed so as to add the solid phase and the second antibody simultaneously to the mixture obtained in the step (A).

11. The method according to claim 8 or 9, wherein step (B) is performed so as to add the second antibody to the mixture obtained in step (A) after adding the solid phase to the mixture.

12. The method according to claim 8 or 9, wherein step (B) is performed so as to add the solid phase to the mixture obtained in step (A) after adding the second antibody to the mixture.

13. The method according to claim 8 or 9, wherein step (B) is performed so as to add a mixture of the solid phase and the second antibody to the mixture obtained in step (A) after mixing the solid phase and the second antibody.

14. The method according to any one of claims 8 to 13 further comprising a step of washing a mixture obtained in step (B) for removing an unreacted first antibody,
wherein step (C) detects the labelling substance of the first antibody bound to the solid phase via the second antibody and the antigen in the washed mixture.
